# EUROPEAN PATENT APPLICATION

(11) **EP 4 257 059 A1**
(43) Date of publication of application: **11.10.2023**
(21) Application number: 21903293.5
(22) Date of filing: 02.12.2021
(51) Int. Cl.: A61B 10/00, G16H 50/20

(54) **MENSTRUATION-RELATED INFORMATION OUTPUT DEVICE, LEARNING DEVICE, METHOD FOR PRODUCING LEARNING INFORMATION, AND RECORDING MEDIUM**

(30) Priority: 07.12.2020 JP 2020202489
(71) Applicant: Suntory Holdings Limited, Osaka 530-8203 (JP)
(72) Inventor: KANEGAWA, Norimasa, Kyoto 6190284 (JP); KUTSUMI, Yuka, Kyoto 6190284 (JP); ZEIDA, Mitsuhiro, Kyoto 6190284 (JP)
(74) Representative: Vossius & Partner Patentanwälte Rechtsanwälte mbB
(86) International application number: PCT/JP2021/044233
(87) International publication number: WO 2022/124189

(57) **Abstract**

The present invention solves a conventional problem that it is not possible to predict menstruation-related information related to menstruation using abdominal sounds. A menstruation-related information output apparatus includes: a learning information storage unit in which learning information configured using two or more pieces of training data having sound information acquired from abdominal sounds of a user and menstruation-related information related to menstruation is stored; a sound information acquiring unit that acquires sound information from abdominal sounds of the user; a predicting unit that applies the learning information to the sound information acquired by the sound information acquiring unit, thereby acquiring menstruation-related information; and an output unit that outputs the menstruation-related information acquired by the predicting unit. Accordingly, it is possible to predict menstruation-related information using abdominal sounds from the abdomen.

## Description

### Technical Field

The present invention relates to a menstruation-related information output apparatus and the like for acquiring and outputting menstruation-related information related to menstruation.

### Background Art

Conventionally, there exists a technique aimed at making it possible to predict a menstruation date while suppressing a decrease in the prediction precision even when the variation in menstrual cycles is large (see Patent Document 1).

Furthermore, there exists a technique for acquiring measurement information related to the state of vaginal discharge and predicting an ovulation date using the measurement information (see Patent Document 2).

### Citation List

### Patent Document

Patent Document 1: Japanese Patent Application No. 2015-523319
Patent Document 2: JP 2014-64706A

### Summary of Invention

### Technical Problem

However, it is not possible for existing techniques to acquire menstruation-related information related to menstruation using abdominal sounds from the abdomen or the vicinity of the abdomen.

### Solution to Problem

A first aspect of the present invention is directed to a menstruation-related information output apparatus including: a learning information storage unit in which learning information configured using two or more pieces of training data having sound information acquired from abdominal sounds of a user and menstruation-related information related to menstruation is stored; a sound information acquiring unit that acquires sound information from abdominal sounds of the user; a predicting unit that applies the learning information to the sound information acquired by the sound information acquiring unit, thereby acquiring menstruation-related information; and an output unit that outputs the menstruation-related information acquired by the predicting unit.

By utilizing this configuration, it is possible to acquire menstruation-related information using abdominal sounds from the abdomen or the vicinity of the abdomen.

A second aspect of the present invention is directed to the menstruation-related information output apparatus according to the first aspect, wherein the menstruation-related information is menstruation date-related information regarding a relationship with a date related to menstruation.

By utilizing this configuration, it is possible to acquire menstruation-related information regarding a relationship with a date related to menstruation using abdominal sounds.

A third aspect of the present invention is directed to the menstruation-related information output apparatus according to the first aspect, wherein the menstruation-related information is pain information regarding menstrual pain.

By utilizing this configuration, it is possible to acquire pain information regarding menstrual pain using abdominal sounds.

A fourth aspect of the present invention is directed to the menstruation-related information output apparatus according to any one of the first to third aspects, wherein the two or more pieces of training data are constituted by training data having the sound information acquired from abdominal sounds acquired from the abdomen on different days in a menstrual cycle of the user and the menstruation-related information.

By utilizing this configuration, it is possible to acquire menstruation-related information using abdominal sounds.

A fifth aspect of the present invention is directed to the menstruation-related information output apparatus according to any one of the first to fourth aspects, further including: a learning unit that performs learning processing through a machine learning algorithm on the two or more pieces of training data, thereby acquiring learning information that serves as a learning model, wherein the predicting unit performs prediction processing through a machine learning algorithm using the sound information acquired by the sound information acquiring unit and the learning information, thereby acquiring menstruation-related information.

By utilizing this configuration, it is possible to acquire menstruation-related information through a machine learning algorithm using abdominal sounds.

A sixth aspect of the present invention is directed to the menstruation-related information output apparatus according to any one of the first to fifth aspects, wherein the sound information is two or more feature values of abdominal sounds of the user.

By utilizing this configuration, it is possible to acquire feature values of sounds from abdominal sounds from the abdomen or the vicinity of the abdomen, and acquire menstruation-related information using the feature values.

A seventh aspect of the present invention is directed to a learning apparatus including: a sound information acquiring unit that acquires sound information from abdominal sounds of a user; a learning accepting unit that accepts menstruation-related information; a training data configuring unit that configures training data from the sound information and the menstruation-related information; a learning unit that performs learning processing through machine learning on the training data configured by the training data configuring unit, thereby configuring learning information that serves as a learning model; and an accumulating unit that accumulates the learning model.

By utilizing this configuration, it is possible to configure, through a machine learning algorithm, a learning model that can predict menstruation-related information using abdominal sounds.

### Advantageous Effects of Invention

According to the menstruation-related information output apparatus of the present invention, it is possible to predict menstruation-related information using abdominal sounds.

### Brief Description of Drawings

FIG. 1 is a conceptual diagram of an information system Ain Embodiment 1.
FIG. 2 is a block diagram of the information system A in the embodiment.
FIG. 3 is a block diagram of a menstruation-related information output apparatus 2 in the embodiment.
FIG. 4 is a flowchart illustrating an operation example of a learning apparatus 1 in the embodiment.
FIG. 5 is a flowchart illustrating a first example of learning information configuring processing in the embodiment.
FIG. 6 is a flowchart illustrating a second example of the learning information configuring processing in the embodiment.
FIG. 7 is a flowchart illustrating an operation example of the menstruation-related information output apparatus 2 in the embodiment.
FIG. 8 is a flowchart illustrating an example of prediction processing in the embodiment.
FIG. 9 is a flowchart illustrating an operation example of a terminal apparatus 3 in the embodiment.
FIG. 10 is a diagram showing a training data management table in the embodiment.
FIG. 11 is a diagram showing an output example in the embodiment.
FIG. 12 is a diagram showing an output example in the embodiment.
FIG. 13 is a diagram showing an output example in the embodiment.
FIG. 14 is a schematic view of a computer system in the embodiment.
FIG. 15 is a block diagram of the computer system in the embodiment.

### Description of Embodiment

Below, an embodiment of the menstruation-related information output apparatus and the like will be described with reference to the drawings. The constituent elements denoted by the same reference numerals in the embodiments perform similar operations, and thus a description thereof may not be repeated.

### Embodiment 1

In this embodiment, a menstruation-related information output apparatus will be described that applies sound information regarding abdominal sounds of a user to learning information configured using two or more pieces of training data having sound information regarding abdominal sounds of the user and menstruation-related information, and acquires and outputs menstruation-related information.

The abdominal sounds refer to sounds emanating from the abdomen of a user. The abdominal sounds may be considered to include sounds emanating from the vicinity of the abdomen of the user. The abdominal sounds may include gut sounds emanating from the intestines, for example. The abdominal sounds may include sounds emanating due to blood flow in the abdomen (e.g., abdominal aortic sounds) and sounds emanating from organs such as the stomach. The menstruation-related information is information related to menstruation, which will be described later in detail. Also, the learning information is a learning model that is configured by a learning apparatus, an association table described below, or the like, for example. The learning model may also be said to be a classifier, a model, or the like.

Furthermore, in this embodiment, a learning apparatus will be described that performs learning processing through a machine learning algorithm from two or more pieces of training data having sound information regarding abdominal sounds of a user and menstruation-related information, and configures a learning model.

Also, in this embodiment, an information system will be described that includes a learning apparatus, a menstruation-related information output apparatus, and one or more terminal apparatuses.

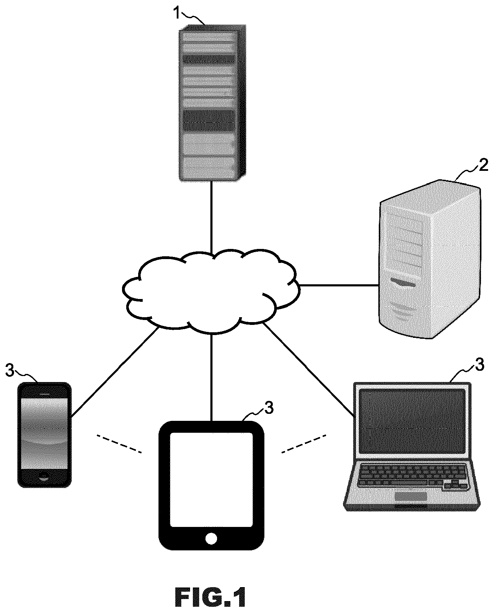

FIG. 1 is a conceptual diagram of an information system Ain this embodiment. The information system A includes a learning apparatus 1, a menstruation-related information output apparatus 2, and one or at least two terminal apparatuses 3.

The learning apparatus 1 is an apparatus that performs learning processing through a machine learning algorithm from two or more pieces of training data having sound information and menstruation-related information, thereby configuring a learning model.

The menstruation-related information output apparatus 2 is an apparatus that acquires and outputs menstruation-related information using abdominal sounds.

The learning apparatus 1 and the menstruation-related information output apparatus 2 are so-called computers, and are servers, for example. The learning apparatus 1 and the menstruation-related information output apparatus 2 are so-called cloud servers, ASP servers, or the like, for example, and there is no limitation on the type thereof. The learning apparatus 1 and the menstruation-related information output apparatus 2 may be stand-alone apparatuses.

Each terminal apparatus 3 is a terminal that is used by a user. The user is a user who wants to acquire menstruation-related information. The terminal apparatus 3 is a terminal for acquiring learning information. The terminal apparatus 3 is a so-called personal computer, tablet device, smartphone, or the like, for example, and there is no limitation on the type thereof.

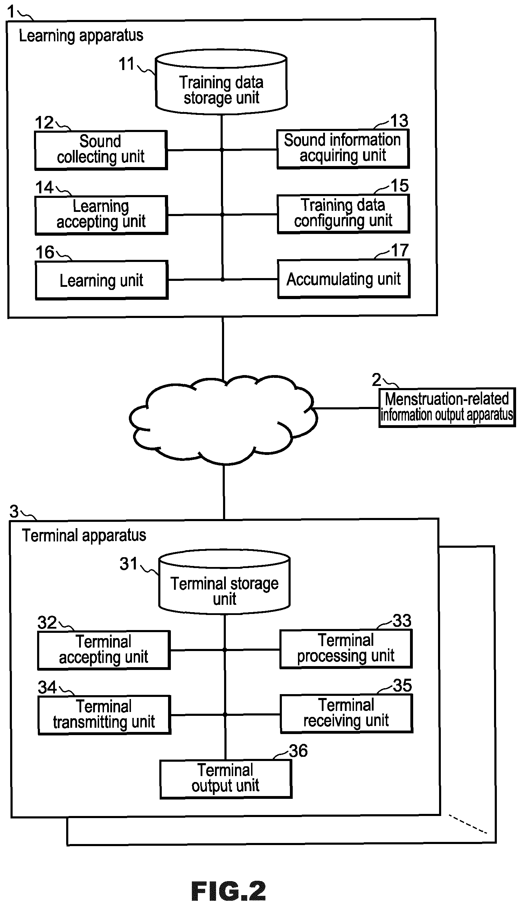

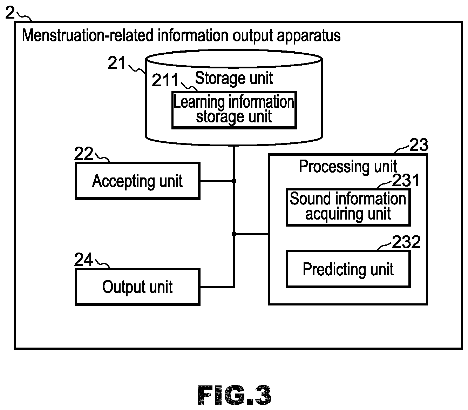

FIG. 2 is a block diagram of the information system A in this embodiment. FIG. 3 is a block diagram of the menstruation-related information output apparatus 2.

The learning apparatus 1 includes a training data storage unit 11, a sound collecting unit 12, a sound information acquiring unit 13, a learning accepting unit 14, a training data configuring unit 15, a learning unit 16, and an accumulating unit 17.

The menstruation-related information output apparatus 2 includes a storage unit 21, an accepting unit 22, a processing unit 23, and an output unit 24. The storage unit 21 includes a learning information storage unit 211. The processing unit 23 includes a sound information acquiring unit 231 and a predicting unit 232.

The terminal apparatus 3 includes a terminal storage unit 31, a terminal accepting unit 32, a terminal processing unit 33, a terminal transmitting unit 34, a terminal receiving unit 35, and a terminal output unit 36.

One or at least two pieces of training data are stored in the training data storage unit 11 constituting the learning apparatus 1. The training data has sound information and menstruation-related information. The sound information refers to information obtained based on abdominal sounds. The sound information may be the recorded abdominal sound data itself, or data obtained by processing or editing the data.

The sound information is a spectrum image in which a result of analysis such as Fourier transform or Fast Fourier transform performed on sound data (which may be processed) obtained by recording abdominal sounds is represented in a predetermined manner, for example. The sound information may be the sound data itself (which may be processed) or data converted to other formats, for example. The sound information may be a set of feature values obtained by performing A/D conversion on abdominal sounds and performing cepstrum analysis on the A/D converted data, for example. The sound information may be a set of feature values obtained by performing A/D conversion on abdominal sounds and performing LPC analysis on the A/D converted data, for example. The sound information is two or more feature values of sounds acquired from abdominal sounds of a user.

The sound collecting unit 12 collects abdominal sounds from the abdomen or the vicinity of the abdomen of a user. The sound collecting unit 12 is a microphone, for example.

The sound information acquiring unit 13 acquires sound information. The sound information is information acquired from abdominal sounds. The sound information acquiring unit 13 acquires sound information for use in prediction processing (described below) for acquiring menstruation-related information, from abdominal sounds. The sound information acquiring unit 13 may acquire sound information from abdominal sounds received from the terminal apparatus 3, or may acquire sound information received from the terminal apparatus 3. The sound information acquiring unit 13 may acquire sound information from abdominal sounds acquired by the sound collecting unit 12.

The sound information acquiring unit 13 performs A/D conversion on abdominal sounds, thereby acquiring sound information, for example. The sound information acquiring unit 13 performs cepstrum analysis on abdominal sounds, thereby acquiring sound information that is a vector of multidimensional feature values, for example. The sound information acquiring unit 13 performs LPC analysis on abdominal sounds, thereby acquiring sound information that is a vector of multidimensional feature values, for example.

The learning accepting unit 14 accepts menstruation-related information. The learning accepting unit 14 typically accepts menstruation-related information that is input by a user. The learning accepting unit 14 typically accepts menstruation-related information in association with a user identifier. The user identifier is information for identifying a user. The user identifier is an ID, an email address, a phone number, or a name, for example.

The learning accepting unit 14 may accept abdominal sounds and menstruation-related information. In this case, the learning apparatus 1 does not have to include the sound collecting unit 12.

The learning accepting unit 14 may accept training data having sound information and menstruation-related information. In this case, the learning apparatus 1 does not have to include the sound collecting unit 12 and the sound information acquiring unit 13.

The training data, the abdominal sounds and the menstruation-related information, or the like accepted by the learning accepting unit 14 is preferably associated with a user identifier.

The menstruation-related information is information related to menstruation. The menstruation-related information is menstruation date-related information or pain information, for example. The menstruation date-related information is information regarding a relationship with a date related to menstruation (e.g., the first day of menstruation, the ovulation date, the last day of menstruation). The menstruation date-related information is information indicating whether or not the first day of menstruation is approaching, information indicating whether or not a person is having a menstrual period, day number information indicating the number of days until the first day of menstruation, day number information indicating the number of days until the ovulation date, or menstrual duration information indicating the length of a menstrual period, for example. The pain information is information regarding pain in next menstruation. The pain information is information indicating whether the pain is weak or strong, or information indicating the level of pain (e.g., one of five levels from 1 to 5, one of ten levels from 1 to 10, etc.), for example.

Furthermore, the accepting in this example is typically receiving from the terminal apparatus 3, but may be a concept that encompasses accepting from a microphone, accepting information input from an input device such as a keyboard, a mouse, or a touch panel, and accepting information read from a recording medium such as an optical disk, a magnetic disk, or a semiconductor memory.

The menstruation-related information may be input by any device such as a touch panel, a keyboard, a mouse, or a menu screen.

The training data configuring unit 15 configures training data from sound information and menstruation-related information. The training data configuring unit 15 configures training data that is a vector having sound information and menstruation-related information, for example. The training data configuring unit 15 configures training data that is a vector in which one or more feature values that are sound information and menstruation-related information are taken as elements, for example. The training data is preferably associated with a user identifier.

The training data configuring unit 15 may acquire other menstruation-related information, using the accepted one or at least two pieces of menstruation-related information. That is to say, the accepted menstruation-related information and the menstruation-related information accumulated in association with sound information do not have to be the same information.

The training data configuring unit 15 acquires menstruation-related information "duration of menstruation", using menstruation-related information indicating "first day of menstruation" and menstruation-related information indicating "last day of menstruation", for example. That is to say, the training data configuring unit 15 acquires date information indicating a date on which menstruation-related information indicating "first day of menstruation" was accepted. The training data configuring unit 15 acquires date information indicating a date on which menstruation-related information indicating "last day of menstruation" was accepted. Then, the training data configuring unit 15 calculates a difference between the two pieces of date information, thereby acquiring menstruation-related information "duration of menstruation". The training data configuring unit 15 may acquire date information from a clock (not shown), or acquire date information received from the terminal apparatus 3. There is no limitation on the method for acquiring the date information.

The training data configuring unit 15 acquires menstruation-related information "day number information indicating the number of days until the first day of menstruation", using menstruation-related information indicating "not having menstrual period" and menstruation-related information indicating "first day of menstruation", for example. That is to say, the training data configuring unit 15 acquires date information indicating a date on which menstruation-related information indicating "not having menstrual period" was accepted. The training data configuring unit 15 acquires date information indicating a date on which menstruation-related information indicating "first day of menstruation" was accepted. Then, the training data configuring unit 15 calculates a difference between the two pieces of date information, thereby acquiring menstruation-related information "day number information indicating the number of days until the first day of menstruation".

The training data configuring unit 15 acquires menstruation-related information "day number information indicating the number of days until the ovulation date", using menstruation-related information indicating "first day of menstruation" and menstruation-related information indicating "not having menstrual period", for example. That is to say, the training data configuring unit 15 acquires date information indicating a date on which menstruation-related information indicating "first day of menstruation" was accepted. The training data configuring unit 15 acquires information indicating the ordinary number of days from the first day of menstruation to the ovulation date, from the storage unit 21. Next, the training data configuring unit 15 calculates date information indicating the ovulation date, using date information corresponding to the first day of menstruation and information indicating the number of days until the ovulation date. Next, the training data configuring unit 15 acquires date information regarding a date on which menstruation-related information was received, for example. Next, the training data configuring unit 15 calculates a difference between the date information indicating the ovulation date and the date information regarding a date on which menstruation-related information was received, thereby acquiring menstruation-related information "day number information indicating the number of days until the ovulation date" that is the number of days corresponding to the difference.

The training data configuring unit 15 accumulates the configured training data in the training data storage unit 11. The training data configuring unit 15 preferably accumulates the configured training data in association with a user identifier. The training data configuring unit 15 preferably accumulates the configured training data in association with date information.

The learning unit 16 acquires learning information using one or at least two pieces of training data.

The learning unit 16 acquires, for each user identifier, learning information that is paired with the user identifier, using one or at least two pieces of training data, for example.

The learning unit 16 acquires learning information using one or at least two pieces of training data, for each type of menstruation-related information (e.g., the number of days until the first day of menstruation or the level of pain), for example.

The learning unit 16 acquires learning information using one or at least two pieces of training data, for each type of menstruation-related information and for each user identifier, for example.

The learning unit 16 performs learning processing through a machine learning algorithm on the two or more pieces of training data, thereby acquiring learning information that serves as a learning model, for example. The learning unit 16 performs learning processing through machine learning on the training data configured by the training data configuring unit 15, thereby configuring learning information that serves as a learning model.

There is no limitation on the machine learning algorithm, and examples thereof include deep learning, decision trees, random forests, SVM, and SVR. For example, various machine learning functions and various other existing libraries, such as the TensorFlow library, fastText, tinySVM, and random forest module of R language, can be used for the machine learning. The module may also be said to be a program, software, a function, a method, or the like.

The two or more pieces of training data are preferably constituted by training data having sound information acquired from abdominal sounds on different days in a menstrual cycle of a user and menstruation-related information.

The learning unit 16 configures an association table, for example. The association table has two or more pieces of association information. The association information may also be said to be training data. The association information is information indicating association between sound information and menstruation-related information. The association information is information indicating association between sound information and one or at least two types of menstruation-related information, for example. The association information is information indicating association between sound information and one or more pieces of menstruation-related information, and other one or more pieces of menstruation-related information, for example.

The association table may exist for each user identifier. The association table may exist for each type of menstruation-related information. The association table may exist for each user identifier and for each type of menstruation-related information.

The accumulating unit 17 accumulates the learning information acquired by the learning unit 16. The accumulating unit 17 accumulates the learning model acquired by the learning unit 16, for example. The accumulating unit 17 may accumulate the learning information in a local recording medium or another apparatus such as the menstruation-related information output apparatus 2.

The accumulating unit 17 accumulates, for each user identifier, the learning information acquired by the learning unit 16 in association with the user identifier, for example. The accumulating unit 17 accumulates, for each type of menstruation-related information, the learning information acquired by the learning unit 16 in association with an identifier of the type of menstruation-related information, for example. The accumulating unit 17 accumulates, for each user identifier and for each type of menstruation-related information, the learning information acquired by the learning unit 16 in association with the user identifier and an identifier of the type, for example. The identifier of the type is "whether or not the first day of menstruation is approaching", "whether or not a person is having a menstrual period", "the number of days until the first day of menstruation", "the number of days until the ovulation date", or "the length of a menstrual period", for example.

Various types of information are stored in the storage unit 21 constituting the menstruation-related information output apparatus 2. The various types of information are learning information, for example.

One or at least two pieces of learning information are stored in the learning information storage unit 211. The learning information is the above-described learning model or association table, for example. The learning information is preferably the information acquired by the learning apparatus 1. The learning information in the learning information storage unit 211 is preferably associated with a user identifier. That is to say, different pieces of learning information are preferably used for respective users. Note that the same learning information may be used for two or more users. The learning information is associated with a user identifier and an identifier of a type of menstruation-related information, for example.

The accepting unit 22 accepts abdominal sounds of a user, for example. The accepting unit 22 accepts sound information acquired from abdominal sounds of a user, for example. The accepting unit 22 accepts abdominal sounds or sound information in association with a user identifier, for example.

The accepting unit 22 accepts an output instruction, for example. The output instruction is an instruction to output menstruation-related information. The output instruction has data of abdominal sounds, for example. The output instruction has sound information, for example. The output instruction preferably contains a user identifier.

The accepting unit 22 receives the abdominal sounds, the sound information, or the output instruction, from the terminal apparatus 3, for example.

The accepting information by the accepting unit 22 is typically receiving from the terminal apparatus 3, but may be a concept that encompasses accepting from a microphone, accepting information input from an input device such as a keyboard, a mouse, or a touch panel, and accepting information read from a recording medium such as an optical disk, a magnetic disk, or a semiconductor memory.

The processing unit 23 performs various types of processing. The various types of processing are processing that is performed by the sound information acquiring unit 231 or the predicting unit 232, for example.

The sound information acquiring unit 231 acquires sound information. The sound information acquiring unit 231 may acquire sound information from the abdominal sounds accepted by the accepting unit 22, or the sound information acquiring unit 231 may acquire the sound information accepted by the accepting unit 22. The sound information acquiring unit 231 has the same function as the sound information acquiring unit 13. The sound information acquiring unit 231 may acquire the sound information accepted by the accepting unit 22.

The predicting unit 232 applies the learning information to the sound information acquired by the sound information acquiring unit 231, thereby acquiring menstruation-related information.

The predicting unit 232 acquires menstruation-related information using the sound information acquired by the sound information acquiring unit 231 and the learning information in the learning information storage unit 211.

The predicting unit 232 gives the sound information acquired by the sound information acquiring unit 231 and the learning information in the learning information storage unit 211 to a prediction module of machine learning, and executes the module, thereby acquiring menstruation-related information, for example. As described above, there is no limitation on the machine learning algorithm, and examples thereof include deep learning, decision trees, random forests, SVM, and SVR. The same applies to the learning processing and the prediction processing.

The predicting unit 232 acquires learning information associated with a user identifier corresponding to the sound information acquired by the sound information acquiring unit 231, from the learning information storage unit 211, and applies the learning information to the sound information acquired by the sound information acquiring unit 231, thereby acquiring menstruation-related information, for example. That is to say, the predicting unit 232 preferably acquires menstruation-related information, using different pieces of learning information for respective users. Note that the predicting unit 232 may acquire menstruation-related information, using the same learning information for two or more users or all users.

The predicting unit 232 acquires learning information associated with an identifier of the type of menstruation-related information that is acquired, from the learning information storage unit 211, and applies the learning information to the sound information acquired by the sound information acquiring unit 231, thereby acquiring menstruation-related information of that type, for example.

The predicting unit 232 acquires an identifier of the type of menstruation-related information that is acquired and learning information associated with the user identifier, from the learning information storage unit 211, and applies the learning information to the sound information acquired by the sound information acquiring unit 231, thereby acquiring menstruation-related information, for example.

The predicting unit 232 performs prediction processing through a machine learning algorithm using the sound information and the learning model, thereby acquiring menstruation-related information, for example.

The predicting unit 232 selects sound information that is the closest to the sound information, from the association table, and acquires menstruation-related information that is paired with the selected sound information, from the association table, for example.

The predicting unit 232 selects two or more pieces of sound information that are close to the sound information acquired by the sound information acquiring unit 231 enough to satisfy a predetermined condition (e.g., with the similarity being greater than or equal to a threshold value) from the association table, acquires two or more pieces of menstruation-related information respectively corresponding to the selected two or more pieces of sound information, from the association table, and acquires one piece of menstruation-related information out of the two or more pieces of menstruation-related information, for example. The predicting unit 232 acquires a representative value (e.g., an average value, a median value, or a value selected by majority vote) of the two or more pieces of menstruation-related information, for example.

The output unit 24 outputs the menstruation-related information acquired by the predicting unit 232. The output in this example is typically transmission to the terminal apparatus 3, but may be a concept that encompasses display on a display screen, projection using a projector, printing by a printer, output of a sound, accumulation in an external recording medium, and delivery of a processing result to another processing apparatus or another program.

Various types of information are stored in the terminal storage unit 31 constituting the terminal apparatus 3. The various types of information are a user identifier, for example. The user identifier may be an ID of the terminal apparatus 3 or the like.

The terminal accepting unit 32 accepts various types of information and instructions. The various types of information and instructions are abdominal sounds, menstruation-related information, or an output instruction, for example. The various types of information and instructions may be input by any device such as a microphone, a touch panel, a keyboard, a mouse, or a menu screen.

The terminal processing unit 33 performs various types of processing. The various types of processing are processing for performing A/D conversion on the abdominal sounds accepted by the terminal accepting unit 32 into data of abdominal sounds suitable for transmission, for example. The various types of processing are processing for changing the instructions and information accepted by the terminal accepting unit 32 into those with a data structure suitable for transmission, for example. The various types of processing are processing for changing the information received by the terminal receiving unit 35 into that with a data structure suitable for transmission, for example.

The terminal transmitting unit 34 transmits various types of information and instructions to the learning apparatus 1 or the menstruation-related information output apparatus 2. The various types of information and instructions are abdominal sounds, menstruation-related information, or an output instruction, for example.

The terminal receiving unit 35 receives various types of information from the menstruation-related information output apparatus 2. The various types of information are menstruation-related information, for example.

The terminal output unit 36 outputs various types of information. The various types of information are menstruation-related information, for example. The terminal output unit 36 preferably outputs menstruation-related information, for each type of menstruation-related information.

The training data storage unit 11, the storage unit 21, the learning information storage unit 211, and the terminal storage unit 31 are preferably non-volatile storage media, but may alternately be realized by volatile storage media.

There is no limitation on the procedure in which information is stored in the training data storage unit 11 and the like. For example, information may be stored in the training data storage unit 11 and the like via a storage medium, information transmitted via a communication line or the like may be stored in the training data storage unit 11 and the like, or information input via an input device may be stored in the training data storage unit 11 and the like.

The sound information acquiring unit 13, the training data configuring unit 15, the learning unit 16, the accumulating unit 17, the processing unit 23, the sound information acquiring unit 231, the predicting unit 232, and the terminal processing unit 33 may be realized typically by processors, memories, or the like. Typically, the processing procedure of the sound information acquiring unit 13 and the like is realized by software, and the software is stored in a storage medium such as a ROM. The processing procedure may be realized by hardware (dedicated circuits). The processors may be CPUs, MPUs, or GPUs, for example, and there is no limitation on the type thereof.

The learning accepting unit 14, the accepting unit 22, and the terminal receiving unit 35 are realized by wireless or wired communication parts, for example.

The output unit 24 and the terminal transmitting unit 34 are realized by wireless or wired communication parts, for example.

The terminal accepting unit 32 may be realized by a device driver for an input device such as a microphone, a touch panel, or a keyboard, control software for a menu screen, or the like.

The terminal output unit 36 may be considered to include or to not include an output device such as a display screen or a speaker. The terminal output unit 36 may be realized by driver software for an output device, a combination of driver software for an output device and the output device, or the like.

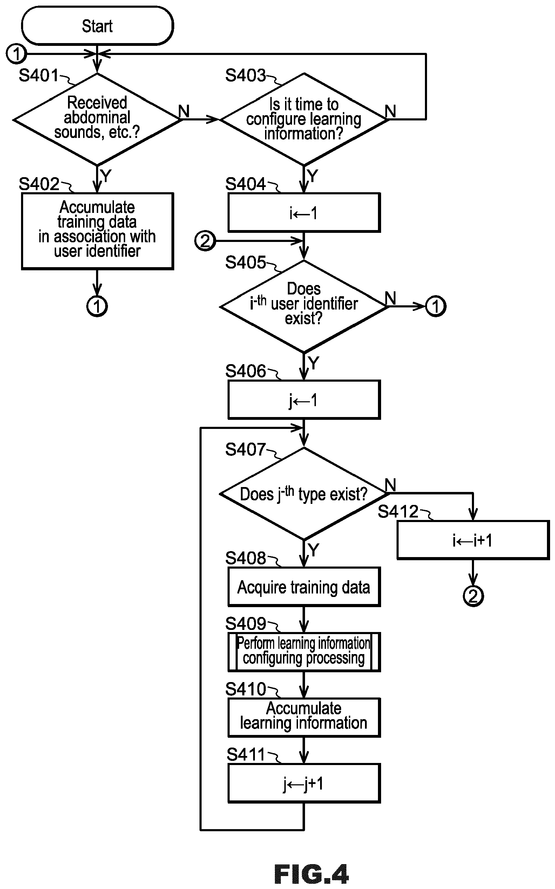

Next, an operation example of the information system A will be described. First, an operation example of the learning apparatus 1 will be described with reference to the flowchart in FIG. 4.

(Step S401) The learning accepting unit 14 determines whether or not it has received abdominal sounds and the like from the terminal apparatus 3. If it has received abdominal sounds and the like, the procedure advances to step S402, or otherwise the procedure advances to step S403. The abdominal sounds and the like are abdominal sounds and menstruation-related information, for example. The abdominal sounds and the like are abdominal sounds, menstruation-related information, and a user identifier, for example. The learning accepting unit 14 does not have to receive abdominal sounds and menstruation-related information together. It is sufficient that abdominal sounds and menstruation-related information are associated with each other.

In this example, the learning accepting unit 14 may receive training data. In this case, the training data configuring unit 15 accumulates the received training data in the training data storage unit 11. The learning accepting unit 14 may receive training data in association with a user identifier.

(Step S402) The sound information acquiring unit 13 acquires sound information from the abdominal sounds received in step S401. Then, the training data configuring unit 15 configures training data having the sound information and the received menstruation-related information. Then, the training data configuring unit 15 accumulates the training data in the training data storage unit 11 in association with a user identifier. The procedure returns to step S401.

(Step S403) The learning unit 16 determines whether or not it is time to configure learning information. If it is time to configure learning information, the procedure advances to step S404, or otherwise the procedure returns to step S401.

The learning unit 16 may determine that it is time to configure learning information, according to an instruction from the terminal apparatus 3. The learning unit 16 may determine that it is time to configure learning information, in the case in which the number of pieces of training data in the training data storage unit 11 is greater than or equal to a threshold value. The learning unit 16 may determine that it is time to configure learning information corresponding to a user identifier, in the case in which the number of pieces of training data corresponding to that user identifier is greater than or equal to a threshold value. The learning unit 16 may determine that it is time to configure learning information, in the case in which training data on days that are dispersed at a predetermined level during a menstrual cycle exists in the training data storage unit 11. The learning unit 16 may determine that it is time to configure learning information, in the case in which days with multiple pieces of training data corresponding to one user identifier during a menstrual cycle (any days in the cycle) satisfy a condition regarding a predetermined level of dispersion. "Predetermined level of dispersion" is dispersion of days in a menstrual cycle, and is that the number of pieces of training data on different days from the first day of menstruation to the first day of next menstruation is greater than or equal to a threshold value (e.g., 15 days or more) or is greater than a threshold value (e.g., more than 18 days), for example.

(Step S404) The learning unit 16 substitutes 1 for a counter i.

(Step S405) The learning unit 16 determines whether or not there is an i^{-th} user identifier for which learning information is to be configured. If there is an i^{-th} user identifier, the procedure advances to step S406, or otherwise the procedure returns to step S401.

(Step S406) The learning unit 16 substitutes 1 for a counter j.

(Step S407) The learning unit 16 determines whether or not there is a j^{-th} type of menstruation-related information for which learning information is to be configured. If there is a j^{-th} type of menstruation-related information, the procedure advances to step S408, or otherwise the procedure advances to step S412.

(Step S408) The learning unit 16 acquires one or more pieces of training data paired with the i^{-th} user identifier and containing the j^{-th} type of menstruation-related information, from the training data storage unit 11.

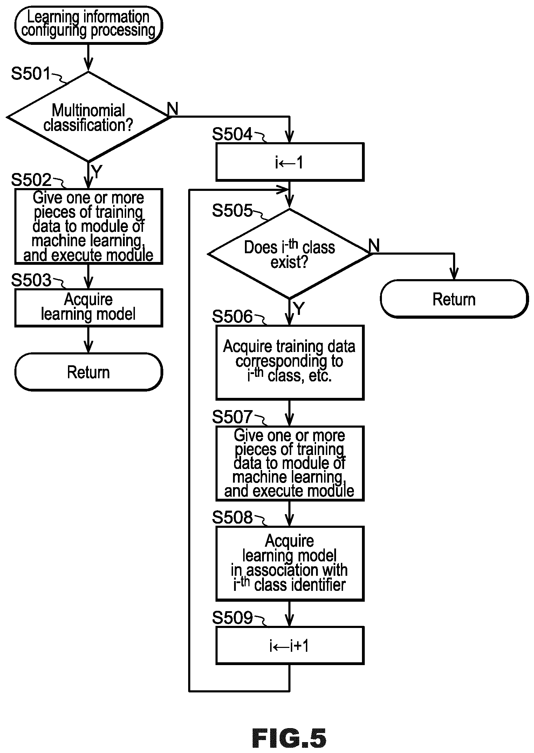

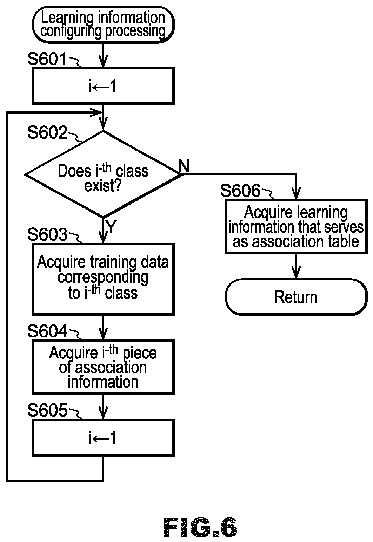

(Step S409) The learning unit 16 configures learning information using the one or more pieces of training data acquired in step S408. An example of the learning information configuring processing will be described later with reference to the flowcharts in FIGS. 5 and 6.

(Step S410) The accumulating unit 17 accumulates the learning information acquired in step S407, in association with the i^{-th} user identifier and a type identifier of the j^{-th} type. The learning information may be accumulated in the learning apparatus 1 or the learning information storage unit 211 of the menstruation-related information output apparatus 2.

(Step S411) The learning unit 16 increments the counter j by 1. The procedure returns to step S407.

(Step S412) The learning unit 16 increments the counter i by 1. The procedure returns to step S405.

In the flowchart in FIG. 4, learning information is configured for each user identifier. However, the same learning information may be configured for two or more users.

Furthermore, in the flowchart in FIG. 4, if these is only one type of menstruation-related information, the learning information is not associated with a type identifier of the type of menstruation-related information.

Also, in the flowchart in FIG. 4, the processing ends at power off or at an interruption of termination processing.

Next, a first example of the learning information configuring processing in step S409 will be described with reference to the flowchart in FIG. 5. The first example shows a case of acquiring learning information that serves as a learning model by performing learning processing through machine learning.

(Step S501) The learning unit 16 determines whether or not to configure learning information that serves as a learning model that performs multinomial classification. If a learning model that performs multinomial classification is to be configured, the procedure advances to step S502, or, if a learning model that performs a learning model that performs binary classification is to be configured, the procedure advances to step S504. Whether the processing to be performed is multinomial classification or binary classification may be predetermined, or may be determined by the learning unit 16 according to the number of pieces of training data to be processed. For example, the learning unit 16 determines to perform "binary classification" in the case in which the number of pieces of training data to be subjected to learning processing is greater than or equal to a threshold value or is greater than a threshold value, and determines to perform "multinomial classification" in the case in which the number of pieces of training data is less than or equal to a threshold value or is less than a threshold value.

(Step S502) The learning unit 16 gives the one or more pieces of training data acquired in step S408 to the learning module of machine learning, and executes the learning module.

(Step S503) The learning unit 16 acquires a learning model as a result of the module being executed in step S502. The procedure returns to the upper-level processing.

(Step S504) The learning unit 16 substitutes 1 for a counter i.

(Step S505) The learning unit 16 determines whether or not there is an i^{-th} class. If there is an i^{-th} class, the procedure advances to step S506, or otherwise the procedure returns to the upper-level processing. The class is candidate data for menstruation-related information. The class is "the first day of menstruation is approaching" or "there is still time until the first day of menstruation", for example.

(Step S506) The learning unit 16 acquires one or more pieces of training data corresponding to the i^{-th} class (positive example) out of the one or more pieces of training data acquired in step S408. The learning unit 16 acquires one or more pieces of training data not corresponding to the i^{-th} class (negative example) out of the one or more pieces of training data acquired in step S406.

(Step S507) The learning unit 16 gives the training data of the positive and negative examples acquired in step S506 to the learning module of machine learning, and executes the learning module.

(Step S508) The learning unit 16 acquires a learning model as a result of the module being executed in step S507, in association with a class identifier of the i^{-th} class.

(Step S509) The learning unit 16 increments the counter i by 1. The procedure returns to step S505.

Next, a second example of the learning information configuring processing in step S409 will be described with reference to the flowchart in FIG. 6. The second example shows a case of acquiring learning information that serves as an association table.

(Step S601) The learning unit 16 substitutes 1 for a counter i.

(Step S602) The learning unit 16 determines whether or not there is an i^{-th} class. If there is an i^{-th} class, the procedure advances to step S603, or otherwise the procedure advances to step S606.

(Step S603) The learning unit 16 acquires one or more pieces of training data corresponding to the i^{-th} class. That is to say, the learning unit 16 acquires one or at least two pieces of sound information corresponding to the i^{-th} class, and acquires a representative value of the one or more pieces of sound information (e.g., a vector in which average values, median values, results of majority vote, or the like, of the feature values are taken as elements), for example. Next, the learning unit 16 acquires training data having the acquired representative value and the i^{-th} class.

(Step S604) The learning unit 16 configures an i^{-th} piece of association information, using the one or more pieces of training data acquired in step S603. The association information is information in which sound information and menstruation-related information (data of a class) are associated with each other.

(Step S605) The learning unit 16 increments the counter i by 1. The procedure returns to step S602.

(Step S606) The learning unit 16 configures an association table having the two or more pieces of association information configured in step S604. The procedure returns to the upper-level processing.

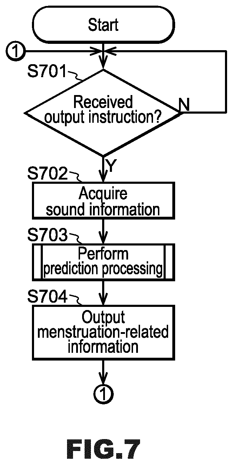

Next, an operation example of the menstruation-related information output apparatus 2 will be described with reference to the flowchart in FIG. 7.

(Step S701) The accepting unit 22 determines whether or not it has received an output instruction from the terminal apparatus 3. If it has received an output instruction, the procedure advances to step S702, or otherwise the procedure returns to step S701. The output instruction contains abdominal sounds and a user identifier, for example. The output instruction may contain sound information and a user identifier.

(Step S702) The sound information acquiring unit 231 acquires sound information from abdominal sounds contained in the output instruction received in step S701.

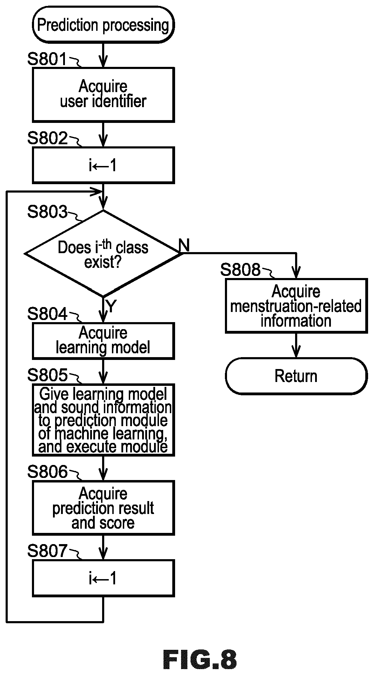

(Step S703) The predicting unit 232 performs prediction processing for acquiring menstruation-related information, using the sound information acquired in step S702. An example of the prediction processing will be described later with reference to the flowchart in FIG. 8.

(Step S704) The output unit 24 transmits the menstruation-related information acquired in step S703, to the terminal apparatus 3. The procedure returns to step S701.

Next, a first example of the prediction processing in step S703 will be described with reference to the flowchart in FIG. 8.

(Step S801) The predicting unit 232 acquires a user identifier corresponding to the received abdominal sounds.

(Step S802) The predicting unit 232 substitutes 1 for a counter i.

(Step S803) The predicting unit 232 determines whether or not there is an i^{-th} class. If there is an i^{-th} class, the procedure advances to step S804, or otherwise the procedure advances to step S808.

(Step S804) The predicting unit 232 acquires a learning model corresponding to the user identifier acquired in step S801 and the i^{-th} class, from the learning information storage unit 211.

(Step S805) The predicting unit 232 gives the learning model acquired in step S805 and the sound information acquired in step S702 to a module that performs prediction processing through machine learning, and executes the module.

(Step S806) The predicting unit 232 acquires a prediction result and a score that are results obtained by executing the module in step S805. The prediction result in this case is information indicating whether or not belonging to the i^{-th} class.

(Step S807) The predicting unit 232 increments the counter i by 1. The procedure returns to step S803.

(Step S808) The predicting unit 232 acquires menstruation-related information using the prediction result and the score acquired in step S806. The procedure returns to the upper-level processing.

The predicting unit 232 acquires a class identifier of a class with the highest core when the prediction result acquired in step S806 is a result indicating "belonging to the i^{-th} class", as menstruation-related information, for example.

In the flowchart in FIG. 8, the prediction processing may be performed for each type of menstruation-related information. The type of menstruation-related information is information indicating whether or not the first day of menstruation is approaching, information indicating whether or not a person is having a menstrual period, day number information indicating the number of days until the first day of menstruation, day number information indicating the number of days until the ovulation date, menstrual duration information indicating the length of a menstrual period, information indicating whether pain in next menstruation will be mild or severe, or information indicating the level of pain, for example.

Furthermore, in the flowchart in FIG. 8, it is also possible to perform second prediction processing. That is to say, the predicting unit 232 acquires a learning model corresponding to a user identifier corresponding to the received abdominal sound, the learning model being capable of performing multinomial classification, from the learning information storage unit 211. Next, the predicting unit 232 gives the learning model and the sound information acquired in step S702 to the module that performs prediction processing through machine learning, and executes the module, thereby acquiring menstruation-related information.

Furthermore, in the flowchart in FIG. 8, it is also possible to perform third prediction processing. That is to say, the predicting unit 232 acquires an association table corresponding to a user identifier corresponding to the received abdominal sound, from the learning information storage unit 211. Next, the predicting unit 232 determines sound information (e.g., a vector) that is the closest to the sound information (e.g., a vector) acquired in step S702, from the association table. Next, the predicting unit 232 acquires menstruation-related information that is paired with the closest sound information, from the association table.

In the flowchart in FIG. 8, the predicting unit 232 may perform prediction processing, using the same learning information for two or more users (a learning model capable of performing multinomial classification, a learning model capable of performing binary classification for each class, or an association table).

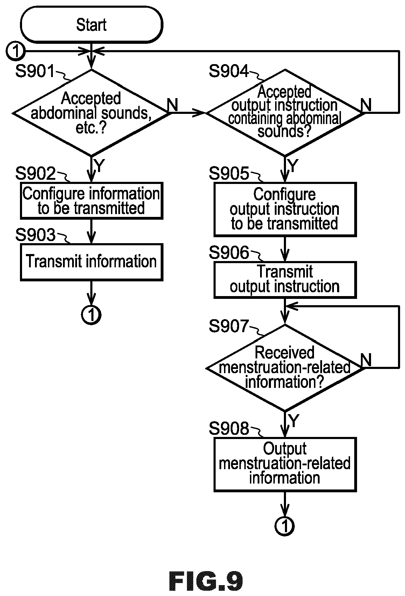

Next, an operation example of the terminal apparatus 3 will be described with reference to the flowchart in FIG. 9.

(Step S901) The terminal accepting unit 32 determines whether or not it has accepted abdominal sounds and the like. If it has accepted abdominal sounds, the procedure advances to step S902, or otherwise the procedure advances to step S904. The abdominal sounds and the like are abdominal sounds and menstruation-related information, for example.

(Step S902) The terminal processing unit 33 configures information that is to be transmitted to the learning apparatus 1, using the abdominal sounds and the like. That is to say, the terminal processing unit 33 acquires a user identifier from the terminal storage unit 31, for example. The terminal processing unit 33 performs A/D conversion on abdominal sounds collected by a microphone. The terminal processing unit 33 configures information that is to be transmitted, the information having data of the A/D converted abdominal sounds, the menstruation-related information, and the user identifier.

(Step S903) The terminal transmitting unit 34 transmits the information configured in step S902, to the learning apparatus 1.

(Step S904) The terminal accepting unit 32 determines whether or not it has accepted an output instruction containing the abdominal sounds. If it has accepted an output instruction, the procedure advances to step S905, or otherwise the procedure returns to step S901.

(Step S905) The terminal processing unit 33 configures an output instruction that is to be transmitted. That is to say, the terminal processing unit 33 acquires a user identifier from the terminal storage unit 31, for example. The terminal processing unit 33 performs A/D conversion on abdominal sounds. The terminal processing unit configures an output instruction having data of the A/D converted abdominal sounds and the user identifier.

In this example, the terminal processing unit 33 may acquire sound information from abdominal sounds, and configure an output instruction having the sound information and the user identifier.

(Step S906) The terminal transmitting unit 34 transmits the output instruction configured in step S905 to the menstruation-related information output apparatus 2.

(Step S907) The terminal receiving unit 35 determines whether or not it has received one or at least two types of menstruation-related information, in response to the transmission of the output instruction in step S906. If it has received menstruation-related information, the procedure advances to step S908, or otherwise the procedure returns to step S907.

(Step S908) The terminal processing unit 33 configures menstruation-related information that is to be output, using the menstruation-related information received in step S907. The terminal output unit 36 outputs the menstruation-related information. The procedure returns to step S901.

In the flowchart in FIG. 9, the processing ends at power off or at an interruption of termination processing.

Hereinafter, specific operation examples of the information system A in this embodiment will be described. FIG. 1 is a conceptual diagram of the information system A.

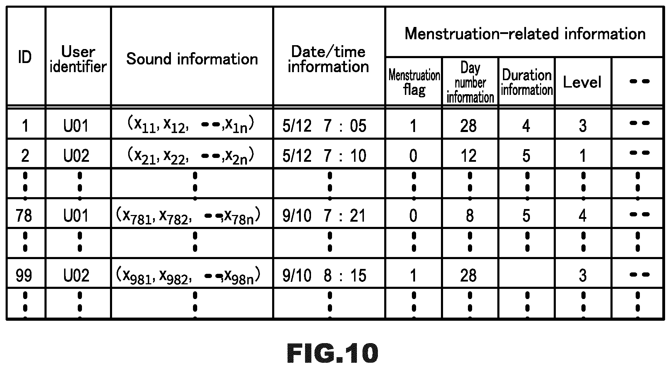

It is assumed that a training data management table having the structure shown in FIG. 10 is stored in the training data storage unit 11 of the learning apparatus 1. The training data management table is a table for managing one or at least two records each having "ID", "user identifier", "sound information", "date/time information", and "menstruation-related information". In this example, "menstruation-related information" has "menstruation flag", "day number information", "duration information", and "level".

In this example, "sound information" is a feature vector, which is a set of two or more feature values acquired from abdominal sounds. "Date/time information" is information indicating the date and time corresponding to the sound information. "Date/time information" may be the date and time at which abdominal sounds were acquired, the date and time at which the learning apparatus 1 received abdominal sounds or sound information, the date and time at which the terminal apparatus 3 transmitted abdominal sounds or sound information, or the like. "Date/time information" includes date information for specifying a date. "Date/time information" may be date information.

"Menstruation flag" is information indicating whether or not a person is having a menstrual period. In this example, the flag is set to "1" in the case in which a person is having a menstrual period, and to "0" in the case in which a person is not having a menstrual period. "Day number information" is information indicating the number of days until the first day of next menstruation. "Duration information" is information indicating the number of days of a menstrual period. "Day number information" is information indicating the duration from the first day to the last day of menstruation in the case in which a person is having a menstrual period, and is information indicating the duration of next menstruation in the case in which a person is not having a menstrual period. "Level" is information indicating the level of menstrual pain, and is a value input by a user.

The training data configuring unit 15 acquires "day number information" as follows, for example. That is to say, the training data configuring unit 15 acquires a user identifier corresponding to the sound information acquired by the sound information acquiring unit 13. The training data configuring unit 15 acquires first date information contained in the date/time information that is paired with the training data paired with the user identifier, the training data containing the menstruation flag "1" and the day number information "28" (training data on the first day of menstruation). The training data configuring unit 15 acquires second date information (second date information < first date information) contained in the date/time information corresponding to the sound information acquired by the sound information acquiring unit 13. Next, the training data configuring unit 15 acquires a difference between the first date information and the second date information as "day number information". In this example, the menstruation cycle is assumed to be 28 days.

Furthermore, the training data configuring unit 15 acquires "duration information" as follows, for example. That is to say, the training data configuring unit 15 acquires a user identifier corresponding to the sound information acquired by the sound information acquiring unit 13. The training data configuring unit 15 acquires first date information contained in the date/time information that is paired with the sound information paired with the user identifier, which is sound information on the first day of menstruation. The training data configuring unit 15 acquires second date information contained in the date/time information that is paired with the user identifier, the date/time information indicating a date after the first date information, indicating a date that is closest to the first date information, and being paired with the menstruation flag "0". The training data configuring unit 15 calculates "duration information" from "duration information = second date information - first date information". Then, the training data configuring unit 15 accumulates "duration information", as an attribute that is paired with the user identifier, that is paired with the date/time information having date information indicating a date before the first date information, and whose "duration information" is NULL. That is to say, the training data configuring unit 15 substitutes the calculated "duration information" for "duration information" contained in training data after the end of the previous menstruation and before the start of the menstruation, once the duration information indicating the duration of menstruation is determined.

In this situation, two specific examples will be described. Specific Example 1 is an example describing the learning processing by the learning apparatus 1. Specific Example 2 is an example describing the prediction processing of menstruation-related information by the menstruation-related information output apparatus 2.

### Specific Example 1

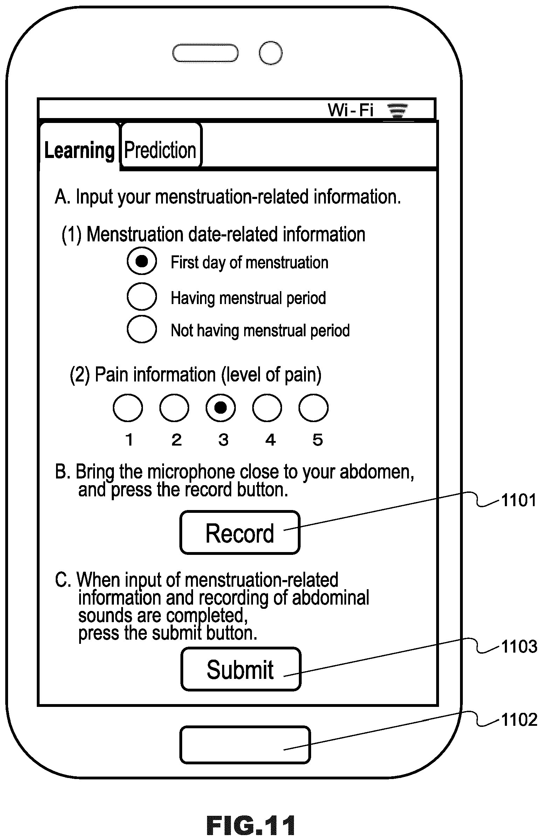

It is assumed that the user identified with "U02" starts an application (hereinafter, referred to as "app" as appropriate) of the terminal apparatus 3 in order to perform training with menstruation-related information. FIG. 11 shows an output example of the app.

It is assumed that the user selects the menstruation date-related information "first day of menstruation" and the pain information "3" on the screen in FIG. 11. Then, the learning accepting unit 14 accepts the menstruation-related information.

Furthermore, it is assumed that the user presses a record button 1101 in FIG. 11, and brings a microphone 1102 of the terminal apparatus 3 close to her abdomen to collect sound information. Then, the terminal accepting unit 32 of the terminal apparatus 3 accepts the abdominal sounds.

Next, it is assumed that the user presses a submit button 1103 in FIG. 11. Then, the terminal processing unit 33 reads the user identifier "U02" of the terminal storage unit 31. Next, the terminal processing unit 33 acquires menstruation-related information "<menstruation date-related information> first day of menstruation <pain information> 3". The terminal processing unit 33 digitizes the abdominal sounds. The terminal processing unit 33 configures information having the menstruation-related information, the abdominal sounds, and the user identifier "U02". Next, the terminal transmitting unit 34 transmits the configured information to the learning apparatus 1.

Next, the learning accepting unit 14 of the learning apparatus 1 receives the menstruation-related information, the abdominal sounds, and the user identifier from the learning apparatus 1.

Next, the training data configuring unit 15 acquires the menstruation flag "1", the day number information "28", and the level "3", from the received menstruation-related information "<menstruation date-related information> first day of menstruation <pain information> 3". Next, the training data configuring unit 15 acquires the date/time information "9/10 8:15" from a clock (not shown). The training data configuring unit 15 acquires various feature values from abdominal sounds, and configures sound information (x₉₈₁, x₉₈₂, ..., x₉₈ₙ). Next, the training data configuring unit 15 configures a record that is to be accumulated in the training data management table. Next, the training data configuring unit 15 accumulates the record in the training data management table. This record is a record "ID = 99" in FIG. 10.

It is assumed that a large number of pieces of training data are accumulated for each user through the above-described training data accumulating processing.

Next, it is assumed that the learning unit 16 configures a learning model for each user and for each type of menstruation-related information as follows. The learning unit 16 may or may not use other types of menstruation-related information, in the case of configuring a learning model for each type of menstruation-related information. For example, in the case of configuring a learning model for outputting the menstruation-related information "level", the learning unit 16 may perform learning processing using training data containing one or more types of menstruation-related information out of other types of menstruation-related information ("menstruation flag", "day number information", and "duration information", in this example), or may perform learning processing using training data not containing other types of menstruation-related information.

That is to say, for example, the learning unit 16 acquires, for each user, all pieces of training data constituted by sound information that is paired with a user identifier of the user and one type of menstruation-related information (e.g., "level") from the training data management table. Next, the learning unit 16 performs learning processing through a machine learning algorithm (e.g., random forests), and configures a learning model in which sound information is taken as input and one type of menstruation-related information (e.g., "level") is taken as output. Next, the accumulating unit 17 accumulates the learning model acquired by the learning unit 16 in a pair with the user identifier.

Furthermore, the learning unit 16 performs the above-described processing for each user and for each of other types of menstruation-related information ("menstruation flag", "day number information", and "duration information"), thereby configuring a learning model for the user and for the menstruation-related information. Next, the accumulating unit 17 accumulates the learning model acquired by the learning unit 16 in a pair with the user identifier.

Through the above-described processing, four learning models each associated with a user identifier of a user and an identifier of a type of menstruation-related information are accumulated.

### Specific Example 2

Next, it is assumed that the user identified with "U02" uses the app as follows in order to predict the number of days until the next menstruation date, the duration of the next menstruation, and the pain level of the next menstruation.

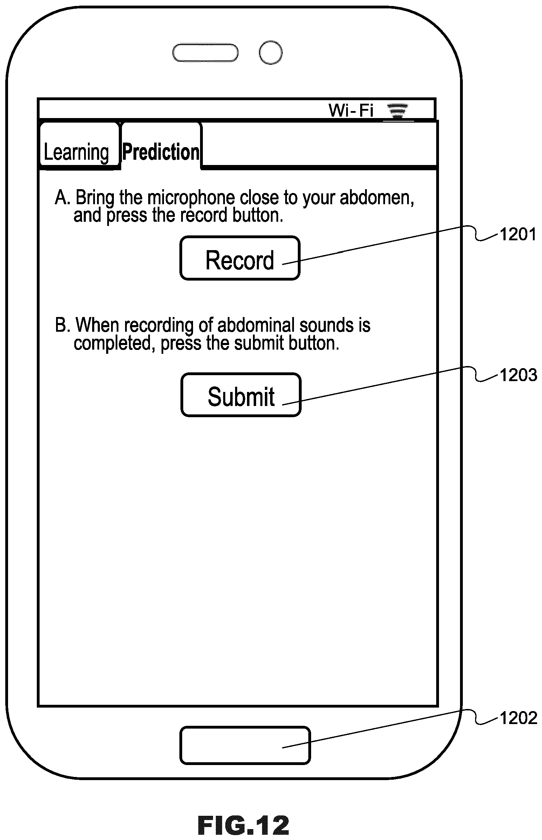

That is to say, it is assumed that the user starts the app of the terminal apparatus 3 in order to predict menstruation-related information. FIG. 12 shows an output example of the app.

Next, it is assumed that the user presses a record button 1201 in FIG. 12, and brings a microphone 1202 of the terminal apparatus 3 close to her abdomen to collect sound information. Then, the terminal accepting unit 32 of the terminal apparatus 3 accepts the abdominal sounds.

Next, it is assumed that the user presses a submit button 1203 in FIG. 12. Then, the terminal processing unit 33 reads the user identifier "U02" of the terminal storage unit 31. Next, the terminal processing unit 33 digitizes the abdominal sounds. The terminal processing unit 33 configures an output instruction having the abdominal sounds and the user identifier "U02". Next, the terminal transmitting unit 34 transmits the output instruction to the menstruation-related information output apparatus 2.

Next, the accepting unit 22 of the menstruation-related information output apparatus 2 receives the output instruction. Then, the sound information acquiring unit 231 acquires a feature vector, which is sound information, from abdominal sounds contained in the received output instruction.

Next, the predicting unit 232 performs prediction processing for acquiring menstruation-related information using the acquired sound information as follows.

That is to say, the predicting unit 232 acquires the user identifier "U02" contained in the output instruction. Next, the predicting unit 232 acquires a learning model corresponding to the user identifier "U02" and the "menstruation flag" from the learning information storage unit 211. Next, it is assumed that the predicting unit 232 gives the learning model and the feature vector, which is sound information, to a module of machine learning (e.g., a random forest module), and executes the module, thereby acquiring the menstruation flag "0".

Furthermore, the predicting unit 232 acquires a learning model corresponding to the user identifier "U02" and the "day number information" from the learning information storage unit 211. Next, it is assumed that the predicting unit 232 gives the learning model and the feature vector, which is sound information, to a module of machine learning (e.g., a deep learning model), and executes the module, thereby acquiring the day number information "3".

Furthermore, the predicting unit 232 acquires a learning model corresponding to the user identifier "U02" and the "duration information" from the learning information storage unit 211. Next, it is assumed that the predicting unit 232 gives the learning model and the feature vector, which is sound information, to a module of machine learning (e.g., an SVM module), and executes the module, thereby acquiring the duration information "4.5".

Also, the predicting unit 232 acquires a learning model corresponding to the user identifier "U02" and the "level" from the learning information storage unit 211. Next, it is assumed that the predicting unit 232 gives the learning model and the feature vector, which is sound information, to a module of machine learning (e.g., a random forest module), and executes the module, thereby acquiring the level "3".

Next, the predicting unit 232 configures menstruation-related information that is to be transmitted, using the menstruation flag "0", the day number information "3", the duration information "4.5", and the level "3". Next, the output unit 24 transmits the configured menstruation-related information to the terminal apparatus 3.

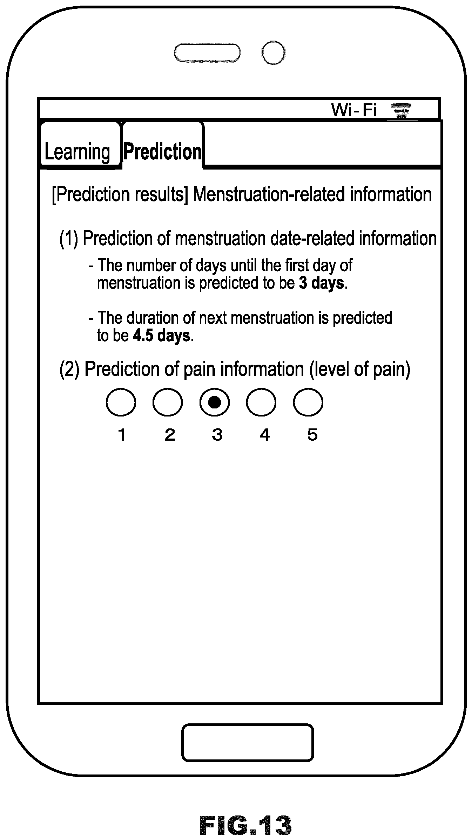

Next, the terminal receiving unit 35 of the terminal apparatus 3 receives the menstruation-related information in response to the transmission of the output instruction. Next, the terminal processing unit 33 configures menstruation-related information that is to be output, using the received menstruation-related information. The terminal output unit 36 outputs the menstruation-related information. FIG. 13 shows an output example thereof.

As described above, according to this embodiment, it is possible to acquire menstruation-related information using abdominal sounds.

In this embodiment, the learning apparatus 1 may use a different machine learning algorithm according to the type of menstruation-related information, in the case of configuring a learning model. The learning apparatus 1 may use a random forest module in the case of configuring a learning model for outputting "menstruation flag", a deep learning model in the case of configuring a learning model for outputting "day number information", and an SVR module in the case of configuring a learning model for outputting "duration information", for example.

Furthermore, in this embodiment, the learning apparatus 1 may be a stand-alone apparatus. The learning apparatus 1 in this case is a learning apparatus 1 including: a sound collecting unit that collects abdominal sounds from the abdomen or the vicinity of the abdomen of a user; a sound information acquiring unit that acquires sound information from the abdominal sounds; a learning accepting unit that accepts menstruation-related information; a training data configuring unit that configures training data from the sound information and the menstruation-related information; a learning unit that performs learning processing through machine learning on the training data configured by the training data configuring unit, thereby configuring learning information that serves as a learning model; and an accumulating unit that accumulates the learning model.

Furthermore, in this embodiment, the menstruation-related information output apparatus 2 may be a stand-alone apparatus. The menstruation-related information output apparatus 2 in this case is a menstruation-related information output apparatus 2 including: a learning information storage unit in which learning information configured using two or more pieces of training data having sound information acquired from abdominal sounds from the abdomen or the vicinity of the abdomen of a user and menstruation-related information related to menstruation is stored; a sound information acquiring unit that acquires sound information for use in prediction processing for acquiring menstruation-related information, from sounds acquired from the abdomen or the vicinity of the abdomen of the user; a predicting unit that applies the learning information to the sound information acquired by the sound information acquiring unit, thereby acquiring menstruation-related information; and an output unit that outputs the menstruation-related information acquired by the predicting unit.

Furthermore, in this embodiment, the menstruation-related information output apparatus 2 may be configured to include the learning apparatus 1.

The processing in this embodiment may be realized by software. The software may be distributed by software downloads or any other suitable method. Furthermore, the software may be distributed in a form where the software is stored in a storage medium such as a CD-ROM. The same applies to other embodiments in this specification. The software that realizes the learning apparatus 1 in this embodiment is the following sort of program. Specifically, this program is a program for causing a computer to function as: a sound information acquiring unit that acquires sound information from abdominal sounds of a user; a learning accepting unit that accepts menstruation-related information; a training data configuring unit that configures training data from the sound information and the menstruation-related information; a learning unit that performs learning processing through machine learning on the training data configured by the training data configuring unit, thereby configuring learning information that serves as a learning model; and an accumulating unit that accumulates the learning model.

Furthermore, the software that realizes the menstruation-related information output apparatus 2 is the following sort of program. Specifically, this program is a program for causing a computer capable of accessing a learning information storage unit in which learning information configured using two or more pieces of training data having sound information acquired from abdominal sounds of a user and menstruation-related information related to menstruation is stored, to function as: a sound information acquiring unit that acquires sound information from abdominal sounds of the user; a predicting unit that applies the learning information to the sound information acquired by the sound information acquiring unit, thereby acquiring menstruation-related information; and an output unit that outputs the menstruation-related information acquired by the predicting unit.

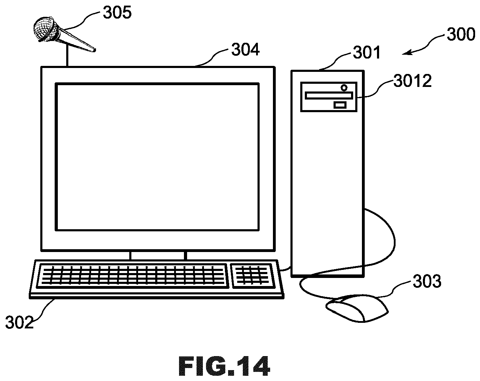

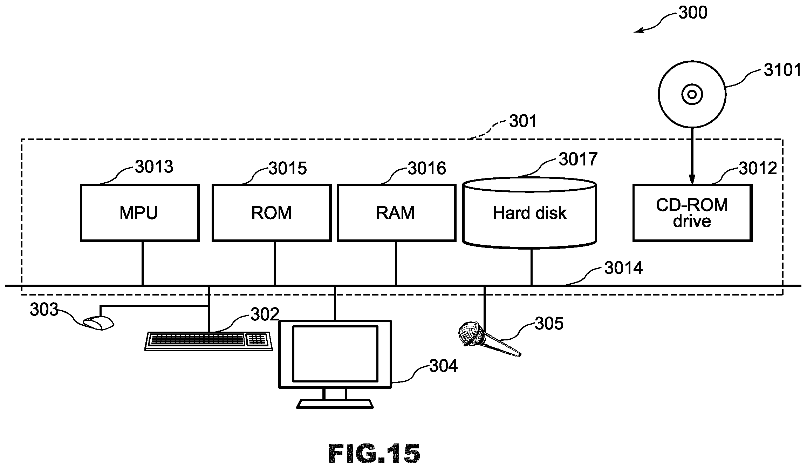

FIG. 14 shows the external appearance of a computer that executes the program described in this specification to realize the learning apparatus 1, the menstruation-related information output apparatus 2, or the terminal apparatus 3 in various embodiments described above. The foregoing embodiments may be realized using computer hardware and a computer program executed thereon. FIG. 14 is a schematic view of a computer system 300. FIG. 15 is a block diagram of the system 300.

In FIG. 14, the computer system 300 includes a computer 301 including a CD-ROM drive, a keyboard 302, a mouse 303, a monitor 304, and a microphone 305.

In FIG. 15, the computer 301 includes, in addition to the CD-ROM drive 3012, an MPU 3013, a bus 3014 connected to the CD-ROM drive 3012 or equivalent, a ROM 3015 in which a program such as a boot up program is stored, a RAM 3016 that is connected to the MPU 3013 and is a memory in which a command of an application program is temporarily stored and a temporary storage area is provided, and a hard disk 3017 in which an application program, a system program, and data are stored. Although not shown, the computer 301 may further include a network card that provides connection to a LAN.

The program for causing the computer system 300 to execute the functions of the learning apparatus 1 and the like in the foregoing embodiment may be stored in a CD-ROM 3101 that is inserted into the CD-ROM drive 3012, and be transmitted to the hard disk 3017. Alternatively, the program may be transmitted via a network (not shown) to the computer 301 and stored in the hard disk 3017. At the time of execution, the program is loaded into the RAM 3016. The program may be loaded from the CD-ROM 3101. Alternately, the program may be loaded directly from a network.

The program does not necessarily have to include, for example, an operating system (OS) or a third party program to cause the computer 301 to execute the functions of the learning apparatus 1 and the like in the foregoing embodiment. The program may only include a command portion to call an appropriate function (module) in a controlled mode and obtain desired results. The manner in which the computer system 300 operates is well known, and thus a detailed description thereof has been omitted.

It should be noted that, in the program, in a step of transmitting information, a step of receiving information, or the like, processing that is performed only by hardware is not included. For example, processing performed by a modem or an interface card in the transmitting step (processing that can be performed only by hardware) is not included.

Furthermore, the computer that executes the program may constituted by a single computer, or constituted by multiple computers. Therefore, centralized processing may be performed, or distributed processing may be performed, respectively. That is to say, the information processing apparatus 5 may constituted by a stand-alone apparatus, or constituted by two or more apparatuses.

Furthermore, in the foregoing embodiment, it will be appreciated that at least two communication parts in one apparatus may be physically realized by one medium.

In the foregoing embodiment, each process may be realized as centralized processing using a single apparatus, or may be realized as distributed processing using multiple apparatuses.

The present invention is not limited to the embodiment set forth herein. Various modifications are possible within the scope of the present invention.

### Industrial Applicability

As described above, the menstruation-related information output apparatus according to the present invention makes it possible to predict menstruation-related information using abdominal sounds from the abdomen or the vicinity of the abdomen, thus rendering this apparatus useful as an apparatus that outputs menstruation-related information and the like.

## Claims

1. A menstruation-related information output apparatus comprising:
a learning information storage unit in which learning information configured using two or more pieces of training data having sound information acquired from abdominal sounds of a user and menstruation-related information related to menstruation is stored;
a sound information acquiring unit that acquires sound information from abdominal sounds of the user;
a predicting unit that applies the learning information to the sound information acquired by the sound information acquiring unit, thereby acquiring menstruation-related information; and
an output unit that outputs the menstruation-related information acquired by the predicting unit.

2. The menstruation-related information output apparatus according to claim 1, wherein the menstruation-related information is menstruation date-related information regarding a relationship with a date related to menstruation.

3. The menstruation-related information output apparatus according to claim 1, wherein the menstruation-related information is pain information regarding menstrual pain.

4. The menstruation-related information output apparatus according to claim 1, wherein the two or more pieces of training data are constituted by training data having the sound information acquired from abdominal sounds acquired from the abdomen on different days in a menstrual cycle of the user and the menstruation-related information.

5. The menstruation-related information output apparatus according to claim 1, further comprising:
a learning unit that performs learning processing through a machine learning algorithm on the two or more pieces of training data, thereby acquiring learning information that serves as a learning model,
wherein the predicting unit performs prediction processing through a machine learning algorithm using the sound information acquired by the sound information acquiring unit and the learning information, thereby acquiring menstruation-related information.

6. The menstruation-related information output apparatus according to claim 1, wherein the sound information is two or more feature values of abdominal sounds of the user.

7. A learning apparatus comprising:
a sound information acquiring unit that acquires sound information from abdominal sounds of a user;
a learning accepting unit that accepts menstruation-related information;
a training data configuring unit that configures training data from the sound information and the menstruation-related information;
a learning unit that performs learning processing through machine learning on the training data configured by the training data configuring unit, thereby configuring learning information that serves as a learning model; and
an accumulating unit that accumulates the learning model.

8. A learning information producing method realized using a sound information acquiring unit, a learning accepting unit, a training data configuring unit, a learning unit, and an accumulating unit, comprising:
a sound information acquiring step of the sound information acquiring unit acquiring sound information from abdominal sounds of a user;
a learning accepting step of the learning accepting unit accepting menstruation-related information;
a training data configuring step of the training data configuring unit configuring training data from the sound information and the menstruation-related information;
a learning step of the learning unit performing learning processing through machine learning on the training data configured in the training data configuring step, thereby configuring learning information that serves as a learning model; and
an accumulating step of the accumulating unit accumulating the learning model.

9. A recording medium on which a program is recorded, the program causing a computer capable of accessing a learning information storage unit in which learning information configured using two or more pieces of training data having sound information acquired from abdominal sounds of a user and menstruation-related information related to menstruation is stored, to function as:
a sound information acquiring unit that acquires sound information from abdominal sounds of the user;
a predicting unit that applies the learning information to the sound information acquired by the sound information acquiring unit, thereby acquiring menstruation-related information; and
an output unit that outputs the menstruation-related information acquired by the predicting unit.

10. A recording medium on which a program is recorded, the program causing a computer to function as:
a sound information acquiring unit that acquires sound information from abdominal sounds of a user;
a learning accepting unit that accepts menstruation-related information;
a training data configuring unit that configures training data from the sound information and the menstruation-related information;
a learning unit that performs learning processing through machine learning on the training data configured by the training data configuring unit, thereby configuring learning information that serves as a learning model; and
an accumulating unit that accumulates the learning model.
